# EUROPEAN PATENT SPECIFICATION

(11) **EP 0 725 606 B1**
(45) Date of publication and mention of the grant of the patent: **01.12.1999**
(21) Application number: 94925601.0
(22) Date of filing: 21.09.1994
(51) Int. Cl.: A61F 2/30

(54) **CAST BONE INGROWTH SURFACE**
GEGOSSENE OBERFLÄCHE FÜR DAS EINWACHSEN VON KNOCHENGEWEBE
SURFACE COULEE POUR INTERPOSITION D'OS

(30) Priority: 29.10.1993 US 146281
(43) Date of publication of application: 14.08.1996
(73) Proprietor: Stryker Technologies Corporation, Kalamazoo, MI 40992 (US)
(72) Inventor: SCHWARTZ, Melvin, M., Point Pleasant, NJ 08742 (US)
(74) Representative: Hammond, Andrew David
(86) International application number: PCT/IB94/00286
(87) International publication number: WO 95/11639

(56) References cited:
- EP-A- 0 255 797
- EP-A- 0 420 542
- DE-A- 3 715 000
- DE-A- 3 844 155
- DE-A- 3 923 418

## Description

### Field of the Invention

This invention relates to an integrally cast tissue ingrowth surface apparatus and a method for casting the same. More particularly, the invention relates to an integrally cast bone or tissue ingrowth surface in a cast metal orthopedic prosthesis.

### Description of the Prior Art

Investment casting or the "lost wax process" has been used for over 50 years in the production of medical and dental implants. The process derives its name from the investment of wax or other suitable mold material and ceramics used to produce an expendable mold for casting metallic implants.

The investment casting process used to prepare orthopedic implants is common to the industry and is used to produce implants from Co-Cr-Mo (Vitallium® [Pfizer Hospital Products Group, Inc., New York, NY]) alloys as well as titanium alloys and stainless steels. Being well suited to the manufacture of the complex shapes typical of many implant designs, investment casting is used extensively to produce components for bone implants or total joint prostheses such as knees and hips.

These prostheses typically consist of metallic and polymeric components where the metallic components rest against bone on one side of the joint and bear against the polymeric component on the other. The bearing surfaces of a total joint have evolved in design to closely mimic the movement of the natural joint, while the bone contacting sides have evolved to assure improved fixation of the implanted prosthesis with the surrounding bone.

Until recently, total joint prostheses were designed for implantation with bone cement. For example, a polymethylmethacrylate (PMMA) grouting agent may be used to secure the prosthesis component against the surrounding bone. Implant surfaces contacting the cement were either cast smooth or with a two dimensional texture intended to improve fixation with the PMMA grout.

Recurrent loosening of these cemented implants, due to loss of support in underlying bone, led to the development of prostheses with three dimensionally porous fixation surfaces which could be used without the PMMA bone cement. These prostheses, instead, rely on fixation via the ingrowth of bone or other connective tissue directly into the prosthesis surfaces, thereby anchoring the prosthesis to the bone.

These three dimensionally textured surfaces are created by bonding a suitable network of material, usually metal of the same composition as the implant, onto the implants fixation surfaces to create a porous coating. The nature of the porosity present in the coating is generally a direct function of the materials and methods used to produce the coating.

Porous surfaces have been created by plasma spraying (United States Patent No. 3,605,123) of fine metallic particles, or by sintering a loosely packed coating of metallic particles (United States Patent No. 4,550,448, British Patent No. 1,316,809), or by diffusion bonding kinked fiber metal pads (United States Patent No. 3,906,550), or overlapping mesh (United States Patent No. 4,636,219).

In another concept, integrally formed ceramic fitted porous areas are formed on the prosthesis. United States Patent No. 4,722,870 discloses a method for investment casting a composite implant which produces a porous metal structure filled with a ceramic (hydroxyapatite). However, this structure cannot be accurately controlled nor can it be spaced a predetermined distance above the outer surface of the implant.

Other United States Patents describe mesh surfaces welded to the implant. Such a mesh is shown in United States Patent No. 3,905,777 to Lacroix, United States Patent No. 4,089,071 to Kalnberz et. al., United States Patent No. 4,261,063 to Blanquaert and United States Patent No. 4,636,219 to Pratt et al. None of these surfaces are integrally cast with the prosthesis.

Each of the aforementioned methods for producing a porous ingrowth surface entails applying a porous network onto the surface of a metallic implant and bonding that network through the application of heat. Plasma spraying employs super heated gases to melt the metal particles to be sprayed. Sintering develops interparticle bonds in a porous coating by exposing the coating and implant metal to temperatures approaching their melting point, while diffusion bonding employs heat and pressure to promote atomic diffusion at the coating implant interface.

Each of these methods has its limitations. Plasma spraying cannot be adequately controlled to achieve a uniform interconnected pore structure in the coating. The temperatures required for sintering have a deleterious effect on the implant material's strength and diffusion bonding develops variations in pore structure and bond quality due to variations in pressure distribution during the coating process. Each of the processes is limited in its achievable pore size by the loss in coating strength which occurs as coating porosity increases.

Particulate porous coatings are also inherently accompanied by a dramatic increase in surface area of metal exposed to body fluids thereby increasing, proportionally, the corrosion products which are released after implantation.

European Patent Application No. 0 230 006 describes a bone implant with a netlike surface covering which contains a large number of perforations for the ingrowth of bony substance and consists of at least two assemblages of elongated crisscrossing elements. To provide satisfactory spaces with precisely predeterminable dimension for newly formed bone tissue, the elongated elements run parallel to the surface of the implant core that lies beneath them, with the attachment of the elements to the core material and maintenance of the distance from the core being ensured by projections.

Clinical reports exist of metal particles becoming loose from bonded coatings or fiber pads becoming detached on revision surgery. Furthermore, bonded coatings inherently develop stress concentrating surface notches at the coating - substrate interface which limit the locations a porous coating can be placed due to strength considerations. By their very nature, bonded coatings require the use of a secondary manufacturing process to affix the coating to the implant surfaces. These processes increase manufacturing costs through added labor, materials, tooling and fixturing.

United States Patent No. 5,108,435, corresponding to EP-A-420542, describes an orthopedic implant comprising a base and a porous surface wherein the base and surface are formed in a one-step casting process. The porous surface is uniformly spaced from the surface of the base.

This prior art document describes a process for preparing a one-piece, cast metal prosthetic bone implant comprising coating a preformed pattern of meltable composition with a ceramic material, removing the meltable composition from the ceramic material by melting the composition to provide a ceramic mold, adding molten metal to the ceramic mold allowing the metal to cool and solidify to form the implant and removing the ceramic material to provide the implant.

### SUMMARY OF THE INVENTION

In one aspect the invention provides a mold according to claim 1. In a second aspect the invention provides a process according to claim 6.

This invention provides a one step process for forming a porous fixation surface (hereafter porous surface) on an implant, for purposes of improved implant fixation to the underlying bone, which is an integral part of the implant. The porous surface is produced by casting a three dimensional grid-like, or lattice structure directly, onto the implant surface.

A further object is to provide a one step process for creating a porous surface on an implant which does not require thermal processing, which may be detrimental to the substrate materials mechanical properties, and does not involve the expense of a secondary coating process.

An object is to allow the porous surface on an implant to be precisely controlled relative to pore shape, pore size, pore size distribution, substrate bonding and coating stress concentrations.

It is yet another object of the invention to allow the pore sizes and shapes to vary and the spacing of the porous surface to be non-uniform.

These and related objects are achieved in the present invention by an implant having a cast metal base member having a first surface designed to rest against a bone after implantation and a tissue ingrowth surface in the form of a cast metal lattice element, composed of a grid-like element, spaced from the base member second surface, and integrally cast with the base member, from the same metal, over at least a part of the second surface thereof. The metal utilized may be "Vitallium", titanium alloy or other suitable biocompatible metallic alloy.

An investment casting technique, wherein a meltable material is coated with a ceramic casting shell, may be utilized to cast the tissue ingrowth porous surface and the base member of the metal orthopedic implant in a single step. As is well known, the meltable material, such as wax, has a melting point lower than the ceramic material. A pattern for the porous surface is formed from the ceramic material, e. g., by injection molding, and inserted in the meltable material die corresponding to the implant. Meltable material is then added to the die partially encapsulating the ceramic pattern and forming a tri-axial matrix network. The combined meltable material ceramic pattern is coated with a ceramic slurry to form a casting shell in the manner well known to the art. The meltable material is then removed from the casting shell by heating. As is well known, the empty casting shell is filled with molten metal and allowed to cool, thereby forming a one piece casting in the form of the pattern corresponding to the orthopedic implant with the lattice integrally cast therewith. Since the preformed ceramic pattern was designed to be semi-exposed the process results in an implant having a three-dimensional porous fixation surface as an integral part of the cast orthopedic product. While wax is preferably used as the meltable material other materials, such as polystyrene, may also be used. The depth of the cavities in the ceramic pattern may be varied to obtain various spacings of the porous surface from the implant.

The process enhances productivity by combining the meltable material injection operations of forming the implant base element and porous fixation surface into one step. This also eliminates any need for additional assembly or joining operations. The process will also attains higher yields during the shell investment cycle of the casting process. Because the internal structure of the meltable material lattice network is embodied in the preformed ceramic, pattern designs are not restricted or inhibited by slurry viscosity or bridging of inadequate shell coating.

Alternatively, the meltable material injection and investment process may be avoided by preparing a mold comprising a ceramic shell and a hollow portion comprising a pattern for the metallic parts of the implant and a ceramic core comprising a pattern for the porous parts of the implant by the method of Direct Shell Production Casting^{TM} (DSPC) (Soligen, Inc., Northridge, CA).

The DSPC method comprises forming a replica of the ceramic shell metallic implant and ceramic pattern for the porous sections of the implant on a computer. The mold is then formed by layers under the control of the computer. After formation of the mold the process proceeds as described above with respect to the wax investment process after removal of the wax from the die.

These and other objects and features of the present invention will become apparent from the following detailed description considered in connection with the accompanying drawings, which disclose several embodiments of the invention. It is to be understood that the drawings are to be used for the purposes of illustration only, and not as a definition of the limits of the invention.

### BRIEF DESCRIPTION OF THE DRAWINGS

Figure 1 is a perspective bottom view of a preformed ceramic pattern for a first embodiment of the porous surface of this invention.
Figure 2 is a bottom plan view of the ceramic pattern of Figure 1.
Figure 3 is an elevational view of the ceramic pattern of Figure 2 along line 3-3 of Figure 2.
Figure 4 is a top plan view of the ceramic pattern of Figure 1.
Figure 5A is schematic sectional view of a preformed ceramic pattern of the first embodiment of this invention.
Figure 5B is schematic sectional view of a meltable material investment mold for use in conjunction with the ceramic pattern of Figure 5A.
Figure 5C is schematic sectional view of the ceramic pattern of Figure 5A emplaced in the meltable material investment mold of Figure 5B.
Figure 6A is schematic sectional view of a meltable casting of an orthopedic implant comprising the base element and porous fixation surface covered by a casting shell after investment with a meltable material and removal from the meltable material die and coating with a ceramic shell.
Figure 6B is schematic sectional view of the casting and ceramic shell of Figure 6A after removal of the meltable material or formation by the DSPC method.
Figure 6C is a view of Figure 6B after molten base metal has been introduced into the ceramic shell of Figure 6B.
Figure 6D is schematic sectional view of the integrally cast orthopedic implant and lattice element of the present invention.
Figure 7 is a partial cross-sectional view of a femoral component of a total hip prosthesis having the porous tissue ingrowth surface in the form of the lattice element integrally cast on the outer surface thereof.
Figure 8 is a schematic sectional view of a second embodiment of the integrally cast orthopedic implant and lattice element of the present invention wherein the porous surface is non-uniformly spaced from the implant.

### DETAILED DESCRIPTION OF THE INVENTION

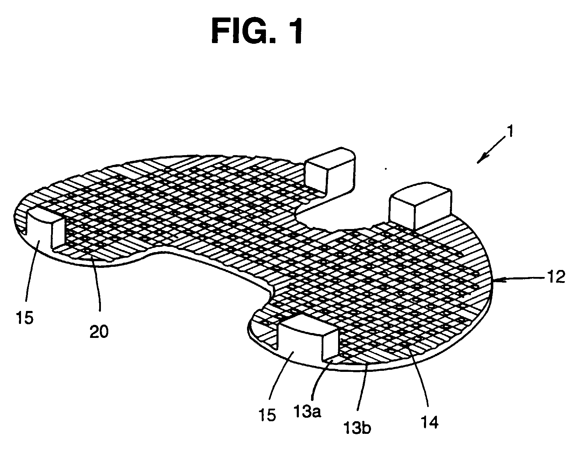

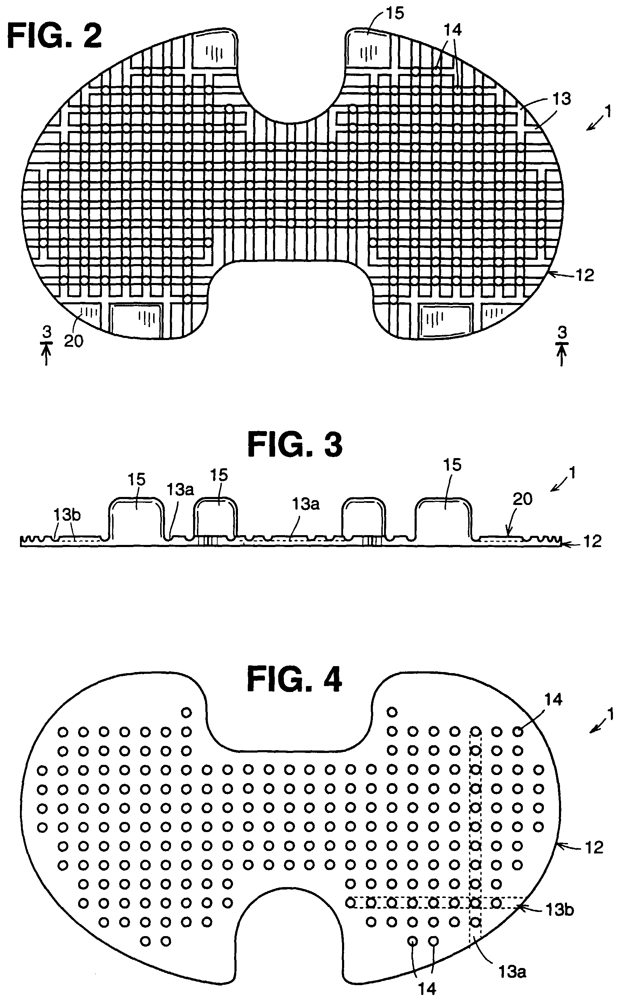

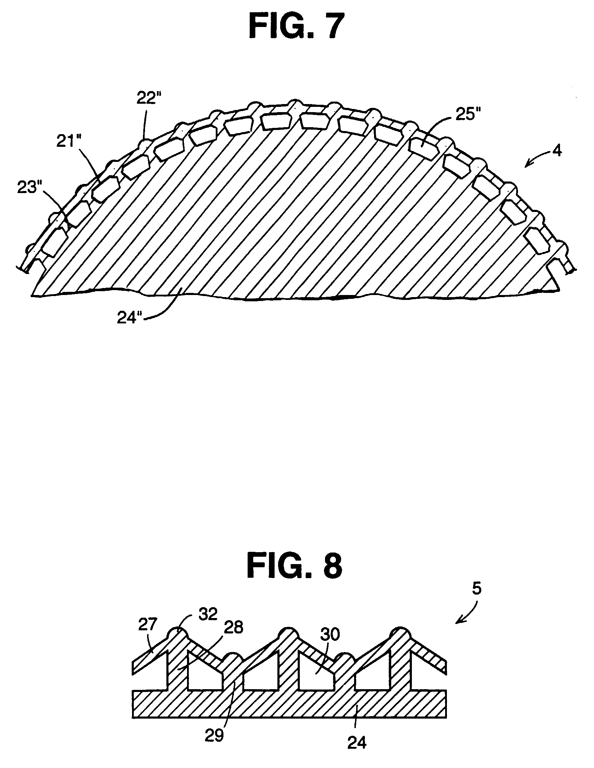

Referring to Figures 1-4 and 7 there is shown a preformed ceramic pattern 1, of the present invention, in the form of a lattice element, for a porous ingrowth surface of an orthopedic implant, such as that for a femoral which is shown in Figure 7. Pattern 1 may be prepared by injection molding in any known manner. Pattern 1 as shown in Figure 1-4 would be useful for a tibial or knee fibial implant. The shape of the pattern may be changed to conform to the shape of implants for other indications such as hip or femoral knee components.

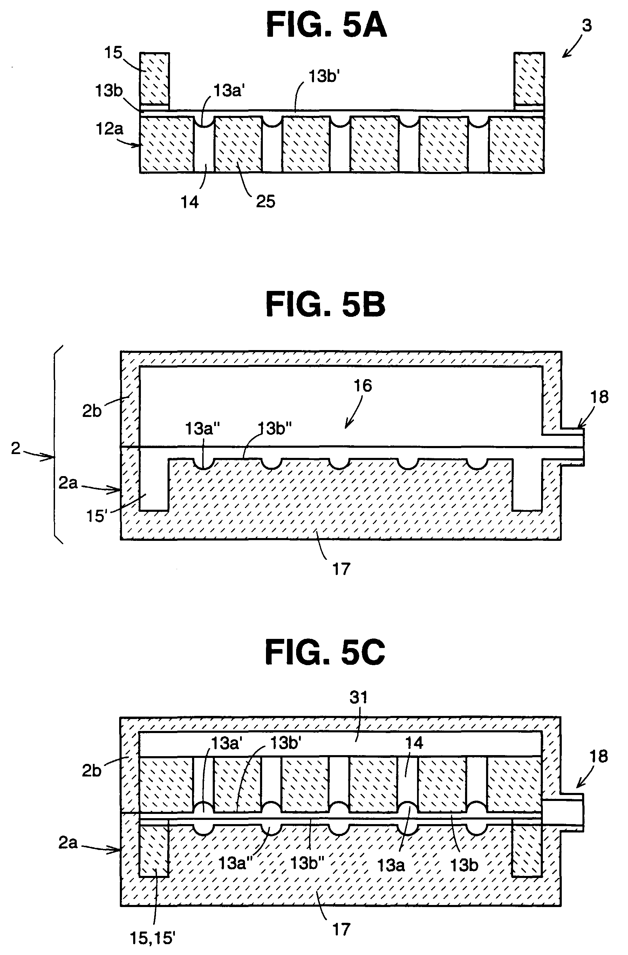

Pattern 1 consists of a flat ceramic sheet 12 comprising transverse, generally perpendicular, grooves 13a and 13b, intersecting at holes 14, corresponding to connectors 23' of the implant in Figure 7, in a first surface 16 of said sheet, which space connectors 13a' and 13b' from the base 24' of the implant. In addition, the pattern comprises projections 15 perpendicular to surface 16, for aligning the pattern in a meltable material investment mold such as is shown schematically in Figure 5B and designated with the numeral 2.

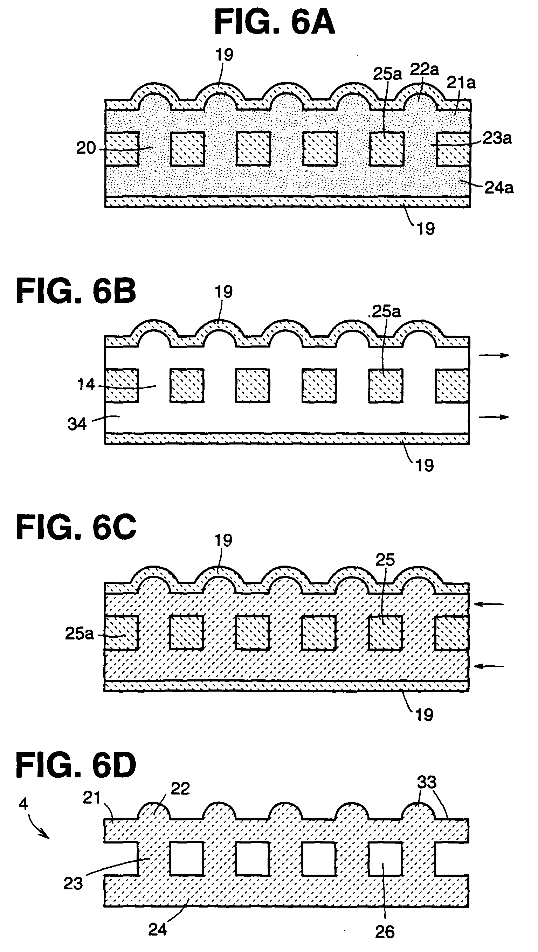

A preferred ceramic pattern for a portion of a porous surface is shown schematically in Figure 5A and designated 3. The pattern, which may be formed by known methods, such as injection molding, comprises a flat ceramic sheet 12a comprising generally perpendicular grooves 13a' and 13b' which intersected holes 14a, through the ceramic sheet 12a. The insert also comprises protrusions 15, perpendicular to the surface of the ceramic sheet 12 which aid in aligning the insert in a meltable composition die such as is shown schematically in Figure 5B and designated 2. The die comprises two releaseably joinable sections, designated 2a and 2b. Section 2a comprises intersecting grooves 13'' and 13b'' which, upon emplacement of the pattern 3 in the die form elongated cavities corresponding to the connectors 21 and 22 of implant 4 in Figure 6D. The second section 2b comprises a cavity 16 a portion 31 of which, that is left over after emplacement of the pattern 3 therein as shown in Figure 5C, corresponds to the base 24 of the implant.

The practice of the invention is best described with reference to Figures 5A-6D, wherein the porous surface pattern 3, shown in Figure 5A, is inserted into section 26 of meltable composition die 2. The meltable material 20 is injected into die 2 through port 18 to fill the cavities 13 formed from grooves 13a', 13b', 13a'' and 13b'', the holes 14 of the ceramic pattern 3 and the cavity 16 of the die. After the cavities comprising grooves 13a', 13b', 13a'' and 13b'', holes 14 and cavity 31 have been filled with the meltable material 20 they form a meltable casting of the implant. The casting is removed from the die and is coated with a colloidal silica binder, to form a shell 19 as shown in Figure 6A. The colloidal silica is selected from the group including refractory powders of zirconia, alumina and silica and is applied to the meltable casting as a slurry. The first coat of the slurry used to form the shell in the investment casting process is critical. A preferred slurry for this first coat is a colloidal silica binder (such as Du Pont's 30% colloidal binder) base with refractory zirconia and silica flours. The viscosity can be varied by adding more or less binder. The dip pattern must be designed to make sure that the one-piece casting is completely and evenly coated. The casting must be vibrated while draining, with air lightly blown over the lattice pattern to break up any air bubbles which might prevent the slurry from bridging the grid openings. With care, it has been found that this technique can be used to produce grid openings of about 50 mm (0.02 inches) and above, With the use of the injection molding process for the meltable material, various pattern shapes, such as square, rectangular or triangular, may be used for the ceramic tissue ingrowth lattice surfaces. With this process, furthermore, the shapes and sizes can be accurately controlled. Thus, various pattern shapes can be fabricated to fit specific implant designs. Furthermore, the potential variability of pore spacing would allow for the use of bone inductive coatings or fillers such as hydroxyapatite to facilitate tissue or bone ingrowth as well as more precise engineering and control of pore structures as required for improved osseo integration or vascularization.

After the initial coat is allowed to dry, the process continues with, additional slurry coats being applied, in the well known manner, as desired, to complete forming the ceramic shell 19 on the meltable material pattern for the porous surface and implant, as shown in Figure 6A. The meltable casting, comprising the intersecting is then removed from the shell by heating in a well known manner. This results in a void being formed within ceramic shell 19, as shown in Figure 6B corresponding to connectors 21a and 22a, spacers 23a of the surface and base 24a, of the implant 4 shown in Figure 6D. A molten metal, such as Vitallium or a tantalum alloy or titanium, is introduced into the void, as shown in Figure. 6C, and allowed to cool. Of course, it is well known that in order to cast titanium, special foundry practices must be followed. Underthose circumstances, cast ingrowth surface of the present invention, comprising titanium, can be produced.

Removal of shell 19, integral one-piece metal casting of an implant 4, comprising a base 24, and a porous fixation surface comprising a lattice element comprising intereseting connectors 21 and 22 connectors 23 to space the lattice element from the base 24 and pores 26 as shown in Figure 6D.

Referring to Figure 7, it can be seen that the integral cast lattice element forming a tissue ingrowth surface comprising connectors 21' and 22', can be easily produced on the outside of a femoral component of a hip prosthesis. This is accomplished by producing a spherical preformed ceramic core that replicates the outer surface 24'' of the hip prosthesis utilizing a meltable material formulation, such as wax or polystyrene, which is suitably flexible and can be wrapped around the outer surface of a hip prosthesis. Such a wax is Yates JW-2® (Yates Manufacturing Co., Chicago, IL 60608) wax.

Alternatively, the mold of Figure 6B may be prepared by the process of Direct Shell Production Casting™ (Soligen, Inc., Northridge, CA.)

In a second embodiment of the invention the porous surface of the implant is not uniformly spaced from the base. As shown, in Figure 8, for an implant designated by the numeral 5, alternate rows of spacers 28 and 29, in the implant have different heights thus resulting in differing displacements of the connectors 27 from the base 29 and varying forms of the pores 30.

In a third embodiment, now shown, alternate spacers, rather than rows of spacers, may have varying heights.

While several examples of the present invention have been described, it is obvious that many changes and modifications may be made thereunto, without departing from the scope of the invention.

## Claims

1. A mold (2, 3) for use in preparing a one-piece, cast metal prosthetic bone implant (4), the implant having a base (24) and a porous ingrowth surface, the porous ingrowth surface having a lattice element (33) with intersecting elongated connecting members (21, 22) spaced from the base by spacing elements (23) that connect the base and the lattice element, the mold comprising
(a) a preformed ceramic pattern (3), the pattern comprising a ceramic sheet (12a) having on a first surface thereof a plurality of intersecting grooves (13a', 13b'), holes (14) at the intersections of said grooves and protrusions (15) from the first surface; and
(b) a meltable composition investment die (2), the die having cavities (15') capable of receiving the protrusions (15) in order to aid alignment of the pattern in the die, a cavity (16) and grooves (13a'', 13b''):
the intersecting grooves (13a', 13b') and the grooves (13a'', 13b'') being of complementary dimension and arrangement such that when the pattern is placed in the die and the protrusions are received in the cavities, the grooves together form elongated cavities that are capable of forming the connecting members, the holes are capable of forming the spacing elements and the cavity is capable of forming the base.

2. A mold as claimed in claim 1 wherein the intersecting grooves are not parallel.

3. A mold as claimed in claim 1 wherein the holes are of equal length.

4. A mold as claimed in claim 1 wherein the holes are of unequal length.

5. A mold as claimed in any one of the preceding claims wherein the meltable composition investment die is a wax investment die.

6. A process for preparing a one-piece, cast metal prosthetic bone implant (4) as defined in claim 1 comprising
adding a meltable composition (20) in molten form to a mold (2, 3) as claimed in claim 1,
allowing the meltable composition to cool and solidify to form a meltable composition casting of the implant,
removing the meltable composition casting containing the preformed ceramic pattern (3) from the investment die (2),
coating the casting containing the preformed ceramic pattern with a ceramic material,
removing the meltable composition from the ceramic material by melting the composition to provide a ceramic mold (19) containing the preformed ceramic pattern,
adding molten metal to the ceramic mold containing the preformed ceramic pattern,
allowing the metal to cool and solidify to form the implant and
removing the ceramic material and the preformed ceramic pattern to provide the implant.

7. A process as claimed in claim 6 wherein the ceramic pattern is removed from the implant by leaching with a base.

8. A process as claimed in claim 6 or 7 wherein the metal is a cobalt-chrome alloy.

9. A process as claimed in any one of claims 6 to 8 wherein the meltable composition is a wax or thermoplastic polymer.

10. A process as claimed in claim 9 wherein the meltable composition is a wax.

11. A process as claimed in claim 9 wherein the meltable composition is a polystyrene.

12. A process as claimed in any one of claims 6 to 11 wherein the preformed ceramic pattern is comprised of a ceramic material selected from fused silica and alumina.

13. A process as claimed in claim 12 wherein the ceramic material is fused silica.

14. A process as claimed in any one of claims 6, 7 and 9 to 13 wherein the metal is selected from Vitallium, titanium, tantalum and alloys thereof.

15. A process as claimed in claim 14 wherein the metal is Vitallium.

16. A process as claimed in claim 14 wherein the metal is titanium or an alloy thereof.

17. A process as claimed in claim 14 wherein the metal is tantalum or an alloy thereof.

18. A process for preparing a ceramic mold for a one-piece, cast metal bone implant as defined in claim 1 comprising
creating the design for the ceramic mold on a computer and
converting the design into the ceramic mold containing patterns of the base and of the spacing elements and the connecting members of the lattice element of the implant under computer control, one layer at a time, by spreading ceramic powder then printing it with a liquid binder and removing excess powder.

19. A preformed ceramic pattern (3) as defined in claim 1.

20. A pattern as claimed in claim 19 which is comprised of a ceramic material selected from fused silica or alumina.

21. A pattern as claimed in claim 20 wherein the ceramic material is fused silica.

## Patentansprüche

1. Form (2, 3) zur Verwendung beim Herstellen eines einstückigen, prothetischen Knochenimplantats (4) aus Metallguß, wobei das Implantat eine Basis (24) und eine poröse Oberfläche zum Einwachsen aufweist, die ein Gitterelement (33) mit sich schneidenden, verlängerten Verbindungsteilen (21, 22) enthält, die von der Basis durch Abstandselemente (23) beabstandet sind, welche die Basis und das Gitterelement verbinden, umfassend
(a) eine vorgeformte, keramische Struktur (3) mit einer dünnen, keramischen Lage (12a), die auf deren einen Oberfläche eine Vielzahl von sich schneidenden Rillen (13a', 13b'), Löcher (14) an den Schnittpunkten der Rillen und Vorsprünge (15) von der ersten Oberfläche aufweist; und
(b) eine Ausschmelz-Matrize (2) aus einer schmelzbaren Verbindung, wobei die Matrize Vertiefungen (15') aufweist, welche die Vorsprünge (15) aufnehmen können, um eine Ausrichtung des Musters in der Matrize zu unterstützen, ein Hohlraum (16) und Rillen (13a'', 13b''); wobei die sich schneidenden Rillen (13a', 13b') und die Rillen (13a'', 13b'') komplementäre Abmessungen und eine Anordnung aufweisen, so daß, wenn die Struktur in der Matrize angeordnet ist und die Vorsprünge in den Vertiefungen aufgenommen sind, die Rillen zusammen langgestreckte Hohlräume ausbilden, die Verbindungsteile bilden können, die Löcher die Abstandselemente und der Hohlraum die Basis bilden können.

2. Form nach Anspruch 1, in der die sich schneidenden Rillen nicht parallel sind.

3. Form nach Anspruch 1, in der die Löcher eine gleiche Länge aufweisen.

4. Form nach Anspruch 1, in der die Löcher eine ungleiche Länge aufweisen.

5. Form nach einem der vorhergehenden Ansprüche, in der die Ausschmelz-Matrize aus einer schmelzbaren Verbindung eine Ausschmelz-Matrize aus Wachs ist.

6. Verfahren zum Herstellen eines einstückigen prothetischen Knochenimplantates (4) aus Metallguß nach Anspruch 1, umfassend das Hinzufügen einer schmelzbaren Verbindung (20) in geschmolzener Form in eine Form (2, 3) nach Anspruch 1;
Zulassen, daß sich die schmelzbare Verbindung abkühlt und fest wird, um ein Implantat-Formgußstück aus einer schmelzbaren Verbindung zu bilden;
Entfernen des die vorgeformte, keramische Struktur (3) enthaltenden Formgußstückes aus einer schmelzbaren Verbindung aus der Ausschmelz-Matrize (2);
Beschichten des die vorgeformte, keramische Struktur enthaltende Formgußstückes mit einem keramischen Werkstoff;
Entfernen der schmelzbaren Verbindung aus dem keramischen Werkstoff durch Schmelzen der Verbindung, um eine keramische Form (19) zu erhalten, welche die vorgeformte, keramische Struktur enthält;
Hinzufügen von geschmolzenem Metall in die die vorgeformte, keramische Struktur enthaltende keramische Form;
Zulassen, daß sich das Metall abkühlt und fest wird, um das Implantat zu bilden und Entfernen des keramischen Werkstoffes und der vorgeformten, keramischen Struktur, um das Implantat bereitzustellen.

7. Verfahren nach Anspruch 6, in welchem die keramische Struktur aus dem Implantat durch Auslaugen mit einer Base entfernt wird.

8. Verfahren nach Anspruch 6 oder 7, wobei das Metall eine Kobalt-Chrom-Legierung ist.

9. Verfahren nach einem der Ansprüche 6 bis 8, wobei die schmelzbare Verbindung ein Wachs oder thermoplastisches Polymer ist.

10. Verfahren nach Anspruch 9, wobei die schmelzbare Verbindung ein Wachs ist.

11. Verfahren nach Anspruch 9, wobei die schmelzbare Verbindung ein Polystyrol ist.

12. Verfahren nach einem der Ansprüche 6 bis 11, wobei die vorgeformte, keramische Struktur aus einem keramischen Werkstoff besteht, ausgewählt aus geschmolzener Kieselerde und Aluminiumoxid.

13. Verfahren nach Anspruch 12, in dem der keramische Werkstoff Quarzglas ist.

14. Verfahren nach einem der Ansprüche 6, 7 und 9 bis 13, wobei das Metall ausgewählt ist aus Vitallium, Titan, Tantal und deren Legierungen.

15. Verfahren nach Anspruch 14,wobei das Metall Vitallium ist.

16. Verfahren nach Anspruch 14, wobei das Metall Titan oder eine Legierung davon ist.

17. Verfahren nach Anspruch 14, wobei das Metall Tantal oder eine Legierung davon ist.

18. Verfahren zum Herstellen einer keramischen Form für ein einstückiges Knochenimplantat aus Metallguß nach Anspruch 1, umfassend das Kreieren der Gestaltung für die keramische Form an einem Computer; und durch Computer kontrollierte lagenweise Umsetzung der Gestaltung in die keramische Form, die Strukturen der Basis und der Abstandselemente sowie der Verbindungsteile des Gitterelements des Implantates enthält, durch Auftragen von keramischem Pulver und anschließendem Bedrucken mit einem flüssigen Bindemittel und Entfernen überschüssigen Pulvers.

19. Vorgeformte, keramische Struktur (3) nach Anspruch 1.

20. Struktur nach Anspruch 19, bestehend aus einem keramischen Werkstoff besteht, ausgewählt aus geschmolzender Kieselerde oder Aluminiumoxid.

21. Struktur nach Anspruch 20, wobei der keramische Werkstoff geschmolzene Kieselerde ist.

## Revendications

1. Moule (2, 3) servant à la préparation d'un implant osseux prothétique monobloc en métal coulé (4), ledit implant étant doté d'une base (24) et d'une surface poreuse d'interposition, laquelle surface poreuse d'interposition présente un élément quadrillé (33) avec des éléments de raccordement allongés se croisant (21, 22) séparés de la base par des éléments entretoise (23) reliant la base à l'élément quadrillé,
le moule comprenant
(a) un modèle préformé en céramique (3), ledit modèle comprenant une plaque de céramique (12a) présentant, sur une première face, une pluralité de rainures croisées (13a', 13b'), des trous (14) aux intersections desdites rainures et des protubérances (15) débordant de ladite première face ainsi que
(b) un moule de coulée pour mélange fusible (2), ledit moule présentant des cavités (15') aptes à recevoir les protubérances (15) de manière à faciliter l'alignement du modèle dans le moule, une cavité (16) et des rainures (13a'', 13b'') ;
les rainures croisées (13a', 13b') et les rainures (13a'', 13b'') présentant des dimensions et un agencement complémentaires de sorte que, lorsque le modèle est placé dans le moule et que les protubérances s'engagent dans les cavités, les rainures forment ensemble des cavités allongées aptes à constituer les éléments de raccordement, les trous sont aptes à constituer les éléments entretoise et la cavité est apte à constituer la base.

2. Moule selon la revendication 1, caractérisé en ce que les rainures croisées ne sont pas parallèles.

3. Moule selon la revendication 1, caractérisé en ce que les trous sont de longueur identique.

4. Moule selon la revendication 1, caractérisé en ce que les trous sont de longueur inégale.

5. Moule selon l'une quelconque des revendications précédentes, caractérisé en ce que le moule de coulée pour mélange fusible est un moule de coulée à cire perdue.

6. Procédé pour préparer un implant osseux prothétique monobloc en métal coulé (4) selon la revendication 1, comprenant les étapes consistant à
ajouter un mélange fusible (20) sous forme fondue dans un moule (2, 3) selon la revendication 1,
laisser le mélange fusible refroidir et se solidifier afin de former un moulage en mélange fusible de l'implant,
retirer le moulage en mélange fusible contenant le modèle préformé en céramique (3) du moule de coulée (2),
doter le moulage contenant le modèle préformé en céramique d'un revêtement en matériau céramique,
retirer le mélange fusible du matériau céramique en faisant fondre le mélange pour obtenir un moule en céramique (19) contenant le modèle préformé en céramique,
ajouter du métal coulé dans le moule en céramique contenant le modèle préformé en céramique,
laisser le métal refroidir et se solidifier afin d'obtenir l'implant et
retirer le matériau céramique ainsi que le modèle préformé en céramique afin de dégager l'implant.

7. Procédé selon la revendication 6, caractérisé en ce que le modèle préformé en céramique est dissocié de l'implant par lessivage avec une base.

8. Procédé selon la revendication 6 ou 7, caractérisé en ce que le métal est un alliage chrome-cobalt.

9. Procédé selon l'une quelconque des revendications 6 à 8, caractérisé en ce que le mélange fusible est une cire ou un polymère thermoplastique.

10. Procédé selon la revendication 9, caractérisé en ce que le mélange fusible est une cire.

11. Procédé selon la revendication 9, caractérisé en ce que le mélange fusible est un polystyrène.

12. Procédé selon l'une quelconque des revendications 6 à 11, caractérisé en ce que le modèle préformé en céramique est réalisé dans un matériau céramique pouvant être du verre de silice ou de l'alumine.

13. Procédé selon la revendication 12, caractérisé en ce que le matériau céramique consiste en du verre de silice.

14. Procédé selon l'une quelconque des revendications 6 ou 7, et 9 à 13, caractérisé en ce que le métal est du Vitallium, du titane, du tantale ou des alliages de ces derniers.

15. Procédé selon la revendication 14, caractérisé en ce que le métal est du Vitallium.

16. Procédé selon la revendication 14, caractérisé en ce que le métal est du titane ou un alliage de titane.

17. Procédé selon la revendication 14, caractérisé en ce que le métal est du tantale ou un alliage de tantale.

18. Procédé de préparation d'un moule en céramique pour un implant osseux prothétique monobloc en métal coulé tel que défini dans la revendication 1, comprenant les étapes consistant à
concevoir le modèle de moule en céramique sur ordinateur et à
transformer, sous contrôle informatique, ce modèle en un véritable moule en céramique contenant les modèles de la base et des éléments entretoise ainsi que les éléments de raccordement de l'élément quadrillé de l'implant, et ce couche par couche, en dispersant de la poudre céramique, puis en comprimant cette dernière avec un liant liquide et en retirant l'excès de poudre.

19. Modèle préformé en céramique (3) selon la revendication 1.

20. Modèle selon la revendication 19, comprenant un matériau céramique consistant en du verre de silice ou de l'alumine.

21. Modèle selon la revendication 20, caractérisée en ce que le matériau céramique est du verre de silice.
